# NOUVEAU FASCICULE DE BREVET EUROPEEN

(11) **EP 0 855 899 B2**
(45) Date de publication et mention de la décision concernant l'opposition: **15.06.2005**
(45) Mention de la délivrance du brevet: 20.06.2001
(21) Numéro de dépôt: 97931832.6
(22) Date de dépôt: 30.06.1997
(51) Int. Cl.: A61K 7/06

(54) **COMPOSITION COSMETIQUE PRESSURISEE ET MOUSSE RESULTANTE**
VERDICHTETES KOSMETISCHES PRÄPARAT UND RESULTIERENDER SCHAUM
PRESSURISED COSMETIC COMPOSITION AND RESULTING FOAM

(30) Priorité: 17.07.1996 FR 9608958
(43) Date de publication de la demande: 05.08.1998
(73) Titulaire: L'OREAL, 75008 Paris (FR)
(72) Inventeur: SAMAIN, Henri, F-91570 Bièvres (FR); CRETOIS, Isabelle, F-78220 Viroflay (FR)
(74) Mandataire: Dossmann, Gérard
(86) Numéro de dépôt international: PCT/FR1997/001163
(87) Numéro de publication internationale: WO 1998/003148

(56) Documents cités:
- EP-A- 0 269 641
- EP-A- 0 635 258
- EP-A- 0 723 770
- WO-A-97/33554
- FR-A- 2 709 954
- FR-A- 2 709 955

## Description

L'invention concerne des compositions cosmétiques conditionnées dans un dispositif aérosol en présence d'un agent propulseur et susceptibles de former une mousse comprenant, dans un milieu cosmétiquement acceptable, au moins un polymère fixant, au moins une silicone oxyalkylénée et au moins 30% en poids d'un solvant hydrosoluble ayant un point d'ébullition inférieur à 85°C, les mousses résultantes, ainsi qu'un procédé de traitement cosmétique des fibres kératiniques, telles que les cheveux ou les cils, caractérisé en ce qu'il consiste à appliquer sur les fibres kératiniques ladite mousse résultant de l'expansion à l'air de la composition cosmétique telle que définie ci-dessus.

Des compositions cosmétiques pressurisées dans des dispositifs aérosol, dans des conditions telles qu'elles puissent former une mousse à la sortie du dispositif, sont bien connues et sont utilisées notamment dans le traitement des cheveux et/ou de la peau. On appellera de telles compositions dans la suite de la description "mousse aérosol".

Ces mousses permettent généralement d'obtenir sur les cheveux une bonne répartition des compositions cosmétiques et elles sont en outre d'une utilisation aisée et plus économique quant à la quantité de produit utilisé par rapport aux lotions.

Ces mousses doivent être suffisamment stables pour ne pas se liquéfier rapidement et doivent également disparaître rapidement soit spontanément, soit lors du massage servant à faire pénétrer et/ou à répartir la composition sur les matières kératiniques et plus particulièrement les cheveux.

Les mousses de coiffage et/ou de maintien des cheveux contiennent en général au moins un polymère de préférence anionique, non ionique ou amphotère qui apporte des propriétés de fixation aux cheveux.

Ces polymères sont généralement non moussants ou faiblement moussants et pour obtenir une mousse aérosol il faut donc ajouter un agent moussant et/ou un agent améliorant la qualité de la mousse.

A la différence des laques aérosols classiques, ces compositions présentent l'inconvénient de ne pas permettre la fixation des cheveux dans une forme déjà réalisée. Elles ne permettent pas non plus de mettre en forme la coiffure tout en la fixant simultanément. En effet, ces compositions sont essentiellement aqueuses et leur application mouille les cheveux et ne peut donc maintenir la forme initiale de la coiffure. Pour mettre en forme et fixer la coiffure, on doit donc ensuite effectuer un brushing ou un séchage. De plus, les cheveux sont généralement collés en paquet et la coiffure ne résistent pas aux déformations mécaniques tels que le vent ou les frottements.

On sait que la présence de solvant tels que l'éthanol permet de diminuer le temps de séchage des compositions essentiellement aqueuses. Toutefois, on sait également que la présence d'éthanol en quantité importante ne permet pas d'obtenir des compositions sous forme de mousse à l'aide des tensioactifs ou des polymères moussants classiquement utilisés pour former des mousses.

La présente invention a donc pour but de proposer une composition formant une mousse à la sortie d'un dispositif aérosol qui se répartisse facilement et rapidement et qui permette de fixer une mèche ou une coiffure dans la forme désirée sans brushing ou séchage prolongé.

A cet effet, la demanderesse vient maintenant de découvrir que des compositions comprenant au moins un polymère fixant, au moins une silicone oxyalkylénée et au moins 30% d'un solvant hydrosoluble tel que l'éthanol, forment en présence d'agent propulseur, de façon inattendue et surprenante, des mousses qui conduisent à des propriétés particulièrement intéressantes, notamment une fixation de la coiffure et une amélioration de la tenue de la coiffure dans le temps.

La chevelure a plus de volume, les cheveux sont brillants, et présentent un toucher naturel. Les compositions présentent également l'avantage de ne pas disperser des particules en suspension dans l'atmosphère.

La présente invention a donc pour objet une composition cosmétique à l'état pressurisé dans un dispositif aérosol en présence d'un agent propulseur et formant une mousse à la sortie dudit dispositif, qui est caractérisée par le fait qu'elle comprend, dans un milieu cosmétiquement acceptable, au moins un polymère fixant, au moins une silicone oxyalkylénée comportant au moins un groupe oxyalkyléné de type (-CₓH₂ₓO) dans lequel x varie de 2 à 6 et a et supérieur ou égal à 1, et au moins 30% en poids, exprimé par rapport à la composition exempte d'agent propulseur, d'un solvant hydrosoluble à plus de 5% et ayant un point d'ébullition inférieur à 85°C, comprenant de l'eau dans des concentrations comprises entre 20 et 65% en poids d'eau par rapport au poids total de la composition, système propulseur non compris.

L'invention a également pour objet une composition cosmétique sous forme de moussé, caractérisée par le fait qu'elle résulte de l'expansion à l'air d'une composition telle que définie ci-dessus.

L'invention a également pour objet l'utilisation de ladite silicone oxyalkylénée en tant qu'agent de moussage des compositions pressurisée en aérosol susceptibles de former une mousse et comprenant au moins un polymère fixant et au moins 30% en poids d'un solvant hydrosoluble ayant un point d'ébullition inférieur à 85°C.

D'autres caractéristiques, aspects, objets et avantages de l'invention apparaîtront encore plus clairement à la lecture de la description et des exemples qui suivent.

Les compositions selon l'invention contiennent donc au moins un polymère fixant. Par polymère fixant, on entend tout polymère ayant pour fonction de fixer temporairement la forme de la coiffure. Par pouvoir fixant de la composition contenant un tel polymère fixant, on désigne l'aptitude de cette demière à donner aux cheveux une cohésion telle que la mise en forme initiale de la coiffure sur laquelle la composition est appliquée, soit conservée.

Selon l'invention, on peut utiliser tout polymère fixant connu en soi. On peut utiliser en particulier un polymère fixant choisi parmi les polymères anioniques, cationiques, amphotères, non ioniques et leurs mélanges. Les polymères fixants peuvent être utilisés sous forme solubilisée ou sous forme de dispersions de particules solides ou liquides de polymère.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire moyen en nombre compris entre 500 et environ 5.000.000 et de préférence entre 1000 et 3.000.000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs de formules suivantes : 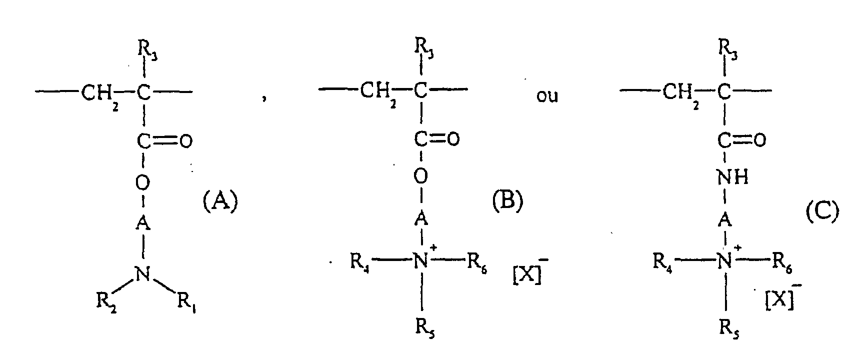 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, Identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer:
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société GIBA GEIGY,
   - les copolymères d'acrylamide et de méthosulfate de méthacryloyloxyéthyltriméthylammonium vendus sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/vinylcaprolactame/vinylpyrrolidone tels que le produit vendu sous la dénomination GAFFIX VC 713 par la société ISP,
   - et les copolymères vinylpyrrolidone/méthacrylamide de diméthylaminopropyle quaternisé tels que notamment le produit vendu sous la dénomination "GAFQUAT HS 100" par la société ISP.
(2) les polysaccharides quaternisés décrits plus particulièrement dans les brevets américains 3.589.578 et 4.031.307 tels que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole;
(4) les chitosanes ou leurs sels; les sels utilisables sont en particulier l'acétate, le lactate, le glutamate, le gluconate ou le pyrrolidone carboxylate de chitosane .
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.

Les polymères fixants anioniques généralement utilisés sont des polymères comportant des groupements dérivés d'acide carboxylique, sulfonique ou phosphorique et ont un poids moléculaire moyen en nombre compris entre environ 500 et 5.000.000.
1) Les groupements carboxyliques sont apportés par des monomères mono ou diacides carboxyliques insaturés tels que ceux répondant à la formule : 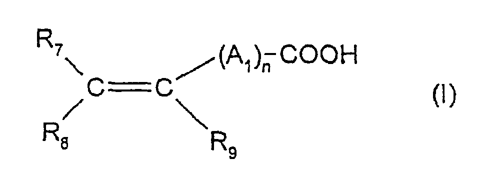
dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle; Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tels que celui vendu par la société ISP sous la dénomination ACRYLIDONE LM et les terpolymères acide méthacrylique/ acrylate d'éthyle/ acrylate de tertiobutyle tels que le produit vendu sous la dénomination LUVIMER 100 P par la société BASF.
C) Les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un autre monomère ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805. Des produits commerçiaux sont notamment ceux vendus sous les dénominations GANTREZ AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées. Ces polymères sont par exemple décrits dans les brevets français 2.350.384 et 2.357.241 de la demanderesse.
E) Les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques tels que ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que les terpolymères acide acrylique / acrylate d'éthyle *l* N-tertiobutylacrylamide vendus notamment sous la dénomination ULTRAHOLD STRONG par la société BASF, les copolymères dérivés d'acide crotonique tels que les terpolymères acétate de vinyle / tertio-butyl benzoate de vinyle *l* acide crotonique et les terpolymères acide crotonique /acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés vendus par exemple sous la dénomination GANTREZ par la société ISP, les copolymères d'acide méthacrylique et de méthacrylate de méthyle vendus sous la dénomination EUDRAGIT L par la société ROHM PHARMA, les copolymères d'acide méthacrylique et d'acrylate d'éthyle vendus sous la dénomination LUVIMER MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol sous la dénomination ARIS-TOFLEX A par la société BASF.

Les polymères fixants anioniques les plus particulièrement préférés sont choisis parmi les copolymères méthylvinyléther / anhydride maléique mono estérifiés vendus sous la dénomination GANTREZ ES 425 par la société ISP, les terpolymères acide acrylique / acrylate d'éthyle / N-tertiobutylacrylamide vendus sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertiobutyl benzoate de vinyle / acide crotonique et les terpolymères acide crotonique / acétate de vinyle /néododécanoate de vinyle vendus sous la dénomination Résine 28-29-30 par la société NATIONAL STARCH, les copolymères d'acide méthacrylique et d'acrylate d'éthyle vendus sous la dénomination LUVIMER MAEX ou MAE par la société BASF, les terpolymères vinylpyrrolidone /acide acrylique /méthacrylate de lauryle vendus sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène a,b-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyaminoamides réticulés et alcoylés partiellement ou totalement dérivant de polyaminoamides de formule générale : 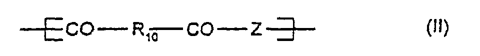 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 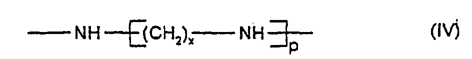 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 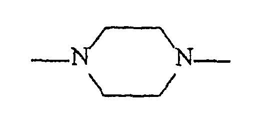
   c) dans les proportions de 0 à 20 moles % le radical -NH-(CH₂)₆-NH-dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique sont par exemple les acides acrylique, méthacrylique, itaconique. Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule : 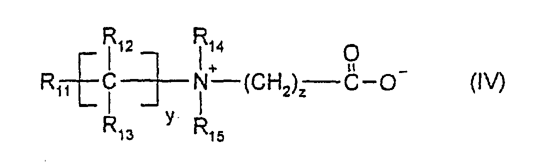 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, un radical méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer les copolymères méthacrylate de méthyle / diméthyl carboxyméthylammonio éthylméthacrylate de méthyle tels que le produit vendu sous la dénomination DIAFORMER Z301 par la société SANDOZ.
(5) les polymères dérivés du chitosane comportant des motifs monomères répondant aux formules suivantes : 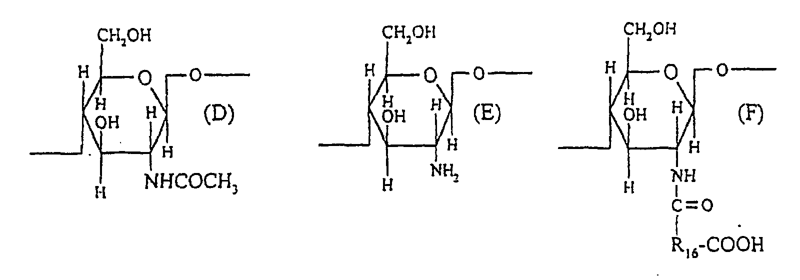 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 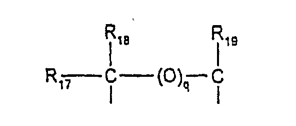 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₇, R₁₈ et R₁₉ étant dans ce cas un atome d'hydrogène ; ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (V) sont par exemple décrits dans le brevet français 1 400 366 : 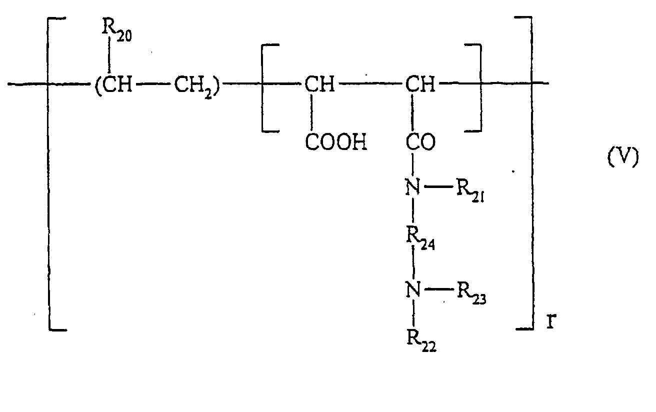 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur en C₁-C₆ tel que méthyle, éthyle, R₂₃ désigne un radical alkyle inférieur en C₁-C₆ tel que méthyle, éthyle ou un radical répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(8) Des polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un radical 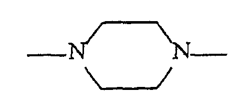 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un radical 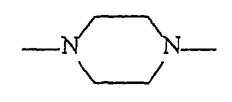 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther / anhydride maléique modifiés partiellement par semiamidification avec une N,N-dialkylaminoalkylamine telle que la N,N-diméthylaminopropylamine ou par semiestérification avec un N, N-dialkylaminoalcanol. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymères méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST;
- les copolymères d'acétate de vinyle et d'ester maléïque par exemple de maléate de dibutyle tels que le produit proposé sous le nom de APPRETAN MB EXTRA par la société HOECHST;
- les homopolymères de chlorure de vinyle tels que les produits proposés sous les noms de GEON 460X45, GEON 480X46 et GEON 577 par la société GOODRICH ;
- les cires de polyéthylène tels que les produits proposés sous les dénominations AQUACER 513 et AQUACER 533 par la société BYK CERA ;
- les cires de polyéthylène/polytétrafluoroéthylène tels que les produits proposés sous les dénominations DREWAX D-3750 par la société DREW AMEROID et WAX DISPERSION WD-1077 par la société R.T. NEWEY;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212 ;
- les copolymères d'acrylonitrile et d'un monomère non ionique choisi par exemple parmi le butadiène et les (méth) acrylates d'alkyle; on peut citer les produits proposés sous les dénominations NIPOL LX 531 B par la société NIPPON ZEON ou ceux proposés sous la dénomination CJ 0601 B par la société ROHM & HAAS;
- les homopolymères de styrène tels que le produit RHODOPAS 5051 proposé par la société RHONE POULENC ;
- les copolymères de styrène et de (méth)acrylate d'alkyle tels que les produits MOWILITH LDM 6911, MOWILITH, DM 611 et MOWILITH LDM 6070 proposés par la société HOECHST, les produits RHODOPAS SD 215 et RHODOPAS DS 910 proposés par la société RHONE POULENC ;
- les copolymères de styrène, de méthacrylate d'alkyle et d'acrylate d'alkyle tels que le produit DAITISOL SPA proposé par la société WACKHERR ;
- les copolymères de styrène et de butadiène tels que les produits RHODOPAS SB 153 et RHODOPAS SB 012 proposés par la société RHONE POULENC ;
- les copolymères de styrène, de butadiène et de vinylpyridine tels que les produits GOODRITE SB VINYLPYRlDINE 2528X10 et GOODRITE SB VINYLPYRIDINE 2508 proposés par la société GOODRICH ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées.
Les gommes de guar non ioniques non modifiées sont par exemple les produits vendus sous la dénomination VIDO-GUM GH 175 par la société UNIPECTINE et sous la dénomination JAGUAR C par la société MEYHALL.
Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-loniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL4H4FD2 par la société AQUALON.

Les radicaux alkyle des polymères non ioniques mentionnés ci-dessus ont, de préférence, de 1 à 6 atomes de carbone.

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640105 et WO 95/00578, EP-A-0582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par:
a) 50 à 90% en poids d'acrylate de tertiobutyle ;
b) 0 à 40% en poids d'acide acrylique ;
c) 5 à 40% en poids de macromère sillconé de formule: 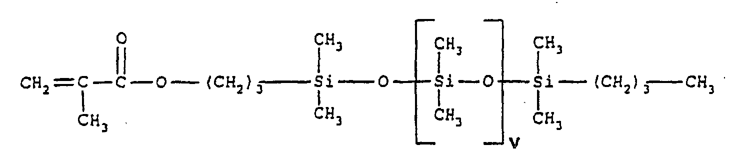
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon la présente invention, les polymères fixants sont de préférence des polymères anioniques.

Les polymères fixants anioniques, amphotères peuvent être si nécessaire neutralisés partiellement ou totalement. Les agents de neutralisation sont par exemple la soude, la potasse, l'amino-2 méthyl-2 propanol-1, la monoéthanolamine, la triéthanolamine ou la triisopropanolamine, les acides minéraux ou organiques tels que l'acide chlorhydrique ou l'acide citrique.

Les compositions selon l'invention comprennent en outre nécessairement au moins une silicone oxyalkylénée. Selon l'invention, on désigne par silicone oxyalkyiénée toute silicone comportant au moins un groupement oxyalkyléné de type (-CₓH₂ₓO)ₐ dans lequel x peut varier de 2 à 6 et a est supérieur ou égal à 1.
Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

Les silicones oxyalkylénées sont par exemple choisies parmi les composés de formules générales (VII), (VIII), (IX), (X) et (XI): 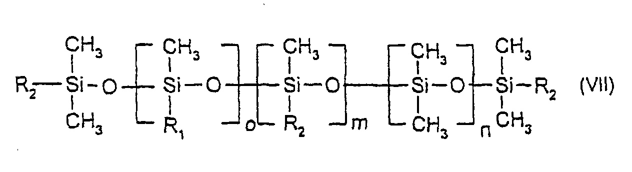 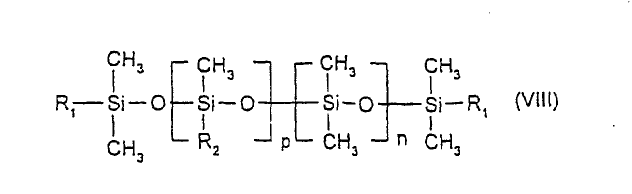 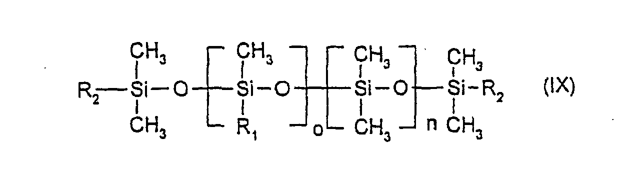 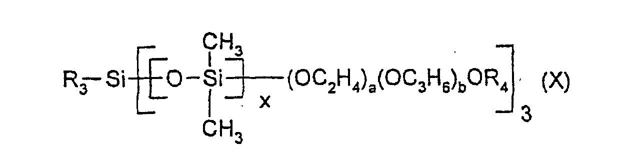 formules dans lesquelles :
- R₁, identique ou différent, représente un radical alkyle, linéaire ou ramifié, en C₁-C₃₀ ou phényle.
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C₁-C₁₂, et de préférence le radical méthyle,
- R₅, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 12 atomes de carbone, un radical hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, -N (CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO (CH₂)_{d}COOM, -OCOCHR₇(CH₂)_{d}COOM, -NHCO(CH₂)_{d}OH, -NH₃Y,
- M, identique ou différent, désigne un atome d'hydrogène, Na, K, Li, NH₄ ou une amine organique,
- R₆ désigne un radical alkyle, linéaire ou ramifié, en C₁-C₃₀,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 1,
- c varie de 0 à 4,
- x varie de 1 à 100,
- Y représente un anion minéral ou organique monovalent tel que halogénure (chlorure, bromure), sulfate, carboxylate (acétate, lactate, citrate).

De préférence, on utilise les silicones oxyalkylénées répondant aux formules générales (VII) ou (VIII). Plus particulièrement, ces formules répondent à au moins une des, et de préférence toutes les, conditions suivantes :
- c est égal à 2 ou 3.
- R₁ désigne le radical méthyle.
- R₅ représente un atome d'hydrogène, un radical méthyle ou un radical acétyle et de préférence un atome d'hydrogène.
- a varie de 1 à 25 et plus particulièrement de 2 à 15.
- b est égal à 0.
- n varie de 0 à 100.
- p varie de 1 à 20

De telles silicones sont par exemple vendues par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DOW CORNING 193, DC 3225 C, Q2-5220, Q2-5354, Q2-5200, par la société RHONE POULENC sous les dénominations SILBIONE HUILE 70646, RHODORSIL HUILE 10634, par la soclété GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WS-L, SILWAX DCA 100, SILTECH AMINE 65, par la société FANNING CORPORATION sous les dénominations FANCORSIL SLA, FANCORSIL LIM1.

Les silicones oxyalkylénées peuvent également être choisies parmi les silicones de formule (XI) suivante :

(XI) ([Y (R₂SiO)ₐ R'₂SiYO][(CₙH₂ₙO)_{b}])_{c}

formule dans laquelle:
- R et R', identiques ou différents, représentent un radical hydrocarboné monovalent,
- n est un nombre entier allant de 2 à 4,
- a est un nombre supérieur ou égal à 4, de préférence compris entre 4 et 200 et encore plus particulièrement entre 4 et 100.
- b est un nombre supérieur ou égal à 4, de préférence compris entre 4 et 200 et encore plus particulièrement entre 5 et 100.
- c est un nombre supérieur ou égal à 4, de préférence compris entre 4 et 1000 et encore plus particulièrement entre 5 et 300.
- Y représente un groupe organique divalent qui est lié à l'atome de silicium adjacent par une liaison carbone-silicium et à un bloc polyoxyalkylène par un atome d'oxygène,
et en particulier:
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids moléculaire moyen en nombre du copolymère bloc pouvant aller de 2.500 à 1.000.000 et de préférence compris entre 3.000 et 200.000 et encore plus particulièrement entre 6.000 et 100.000.

R et R' sont préférentiellement choisis parmi le groupe comprenant les radicaux alkyls linéaires ou ramifiés comme par exemple les radicaux méthyle, éthyle, propyle, butyle, pentyle, hexyle, octyle, décyle, dodécyle, les radicaux aryls comme par exemple phényle, naphtyle, les radicaux aralkyls ou alkylaryls comme par exemple benzyle, phényléthyle, les radicaux tolyle, xylyle.

Y est de préférence -R"-, -R"-CO-, -R"-NHCO-, -R"-NH-CO-NH-R"'-NHCO, -R"-OCONH-R"'-NHCO-, où R" est un groupe alkylène divalent, linéaire ou ramifié en C₁-C₆ comme par exemple l'éthylène, le propylène ou le butylène, linéaire ou ramifié et R''' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical C₄H₈, linéaire ou ramifié.

La préparation des copolymères blocs mis en oeuvre dans le cadre de la présente invention est décrite dans la demande européenne EP 0 492 657 A1, dont l'enseignement est inclus à titre de référence dans la présente description.

Des silicones oxyalkylénées préférées selon l'invention peuvent être choisies parmi celles de formule (XII) :

[C₄H₈ O(C₂H₄O)_{b} (C₃H₆O)_{d}-C₄H₈ - (SiMe₂O)ₐSiMe₂]_{c} (XII)

dans laquelle :
- Me représente méthyle,
- a est un nombre compris entre 4 et 100,
- b et d, identiques ou différents sont des nombres allant de 0 à 100,
- c est un nombre allant de 5 à 300,
- b + d est un nombre allant de 1 à 200.

On choisit de préférence des polymères dont l'unité répétitive est de formule (XII) et dont le rapport en poids siloxane / polyoxyalkylène est d'environ 75/25 et le rapport en poids polyoxyéthylène / polyoxypropylène est d'environ 50/50, des polymères dont le rapport en poids siloxane / polyoxyalkylène est d'environ 35/65 et le rapport en poids polyoxyéthylène / polyoxypropylène est d'environ 100/0, des polymères dont le rapport en poids siloxane / polyoxyalkylène est d'environ 30/70 et le rapport en poids polyoxyéthylène / polyoxypropylène est d'environ 0/100 et plus particulièrement ceux dont le rapport en poids siloxane/polyoxyalkylène est d'environ 20/80 et le rapport en poids polyoxyéthylène / polyoxypropylène est d'environ 65/35.

De tels produits sont par exemple vendus sous la dénomination SILICONE FLUID FZ-2172 par la société OSI.

Les compositions selon l'invention comprennent également, à titre d'élément essentiel, au moins un solvant hydrosoluble présentant un point d'ébullition inférieur à 85°C.

Par solvant hydrosoluble, on entend selon la présente invention un solvant soluble à plus de 5 % en poids dans l'eau.

Le solvant hydrosoluble ayant un point d'ébullition inférieur à 85°C peut être plus particulièrement choisi parmi les alcools en C₂-C₆, de préférence l'éthanol, le tertiobutanol ou l'isopropanol.

Le ou les solvants hydrosolubles ayant un point d'ébullition inférieur à 85°C doivent être présents dans des concentrations supérieure ou égale à 30%, généralement comprises entre 30 et 60%, et de préférence entre 40 et 55%, ces concentrations étant exprimées en poids par rapport au poids total de la composition sans prise en compte de l'agent propulseur.

Le ou les polymères fixants peuvent être présents dans des proportions comprises entre 5 et 40 % en poids par rapport au poids total de la composition sans prise en compte de l'agent propulseur, et de préférence entre 6 et 15 % en poids.

La ou les silicones oxyalkylénées peuvent être présentes dams les compositions selon l'invention dans des proportions comprises entre 0,01 et 10 % en poids par rapport au poids total de la composition sans prise en compte de l'agent propulseur, et de préférence entre 0,05 et 5 % en poids.

Le rapport pondérai solvant hydrosoluble/silicone oxyalkylénée est de préférence compris entre 0,01 et 1.

Selon l'invention, en vue d'obtenir une mousse aérosol, la composition comprend au moins un agent propulseur classique qui peut être choisi parmi les hydrocarbures volatils tels que le n-butane, le propane, l'isobutane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leur mélange.

Le ou les agents propulseurs sont généralement présents dans les compositions selon l'invention dans des concentrations comprises entre 1 et 20% en poids par rapport au poids total de la composition à l'état pressurisée, et de préférence entre 5 et 15% en poids.

Le milieu cosmétiquement acceptable peut contenir en plus du ou des solvants ayant un point d'ébullition Inférieur à 85°C, d'autres solvants cosmétiquement acceptables tels que de l'eau, des polyalcools, des éthers de glycol ou des esters d'acides gras, qui peuvent être utilisés seuls ou en mélange.

On peut citer plus particulièrement les polyalcools tels que le diéthylèneglycol, les éthers de glycol, les monoalkyléthers de glycol ou de diéthylèneglycol ou de propylèneglycol.

Le pH des compositions selon l'invention est généralement compris entre 2 et 10, et en particulier entre 3 et 8. Il peut être ajusté à la valeur choisie au moyen d'agents alcalinisants ou acidifiants habituellement utilisés en cosmétique pour ce type d'application.

Les compositions selon l'invention peuvent encore contenir, des agents épaississants, des agents tensioactifs, des agents conservateurs, des séquestrants, des adoucissants, des parfums, des colorants, des agents modificateurs de viscosité, des agents modificateurs de mousse, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes et des parfums.

Les compositions selon l'invention peuvent également contenir des agents conditionneurs. Ceux-ci peuvent alors être choisis parmi les huiles et les cires naturelles ou synthétiques, les alcools gras, les esters d'alcools polyhydriques, les glycérides, les huiles, les gommes et résines de silicone ou les mélanges de ces différents composés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

L'invention a encore pour objet un procédé de traitement cosmétique des fibres kératiniques, telles que les cheveux ou les cils, caractérisé en ce qu'il consiste à appliquer sur les fibres kératiniques une mousse résultant de l'expansion à l'air de la composition cosmétique telle que définie ci-dessus.

Les compositions cosmétiques selon l'invention peuvent se présenter sous forme de solutions plus ou moins épaissies, de dispersions ou d'émulsions susceptible de former une mousse.

Dans tout ce qui suit ou ce qui précède, les pourcentages sont exprimés, sauf mention contraire, en poids par rapport au poids de la composition ne contenant pas le propulseur.

L'invention va être maintenant plus complètement illustrée à l'aide des exemples suivants qui ne sauraient être considérés comme la limitant aux modes de réalisation décrits. Dans ces exemples, MA signifie matière active.

### EXEMPLE 1

On a préparé une mousse de coiffage (A) de composition suivante :
- Terpolymère acétate de vinyle /p-tertiobutyl benzoate de vinyle/ acide crotonique (polymère fixant) 8 g
- Amino-2 méthyl-2 propanol-1 (qs neutralisation à 100% du polymère fixant) 0,8 g
- Ethanol 49,5 g
- Silicone oxyalkylénée vendue sous la dénomination SILICONE FLUID FZ-2172 par la société OSI 1,05 gMA
- Eau qsp 100 g

### Conditionnement en aérosol :

90g de la composition ci-dessus sont conditionnés dans un récipient aérosol classique en présence de 10g d'un mélange ternaire de n-butane, isobutane et propane, (23/55/22), vendu sous la dénomination de "AEROGAZ 3,2 N" par la société ELF AQUITAINE.

A la sortie du dispositif aérosol, on obtient une belle mousse. On prélève l'équivalent d'une mandarine de mousse que l'on étale rapidement sur les mains puis on applique la mousse à l'aide des mains sur les cheveux lavés et séchés en mettant en forme la chevelure.
La coiffure se fait rapidement et présente une bonne tenue et un bon volume. Les cheveux sont brillants, sans résidus et le toucher est naturel.

### Exemples comparatifs :

On a préparé une composition (B) non conforme à l'Invention en remplaçant dans la composition de l'exemple 1 le copolymère SILICONE FLUID FZ-2172 par la même quantité d'un tensioactif utilisé classiquement comme agent de moussage dans les mousses de l'art antérieur, à savoir le nonylphénol oxyéthyléné à 9 moles d'oxyde d'éthylène (IGEPAL NP 9 de RHONE POULENC).
La composition (B) est ensuite pressurisée de la même manière que la composition (A). A la sortie du dispositif aérosol, on n'obtient pas de mousse.

On a préparé une composition (C) non conforme à l'invention en remplaçant dans la composition de l'exemple 1 le copolymère SILICONE FLUID FZ-2172 par la même quantité d'un polymère utilisé classiquement comme agent de moussage dans les mousses de l'art antérieur, à savoir le copolymère d'aicool vinylique et d'acétate de vinyle (COVOL 9740 de CPC INTERNATIONAL).
La composition (C) est ensuite pressurisée de la même manière que la composition (A). A la sortie du dispositif aérosol, on n'obtient pas de mousse.

On a préparé des compositions (B') et (C'), similaires aux compositions (B) et (C) mais dans lesquelles la teneur en éthanol a été ramenée à 15% environ. Les compositions ont été pressurisées dans un dispositif aérosol comme précédemment. On a obtenu à la sortie du dispositif aérosol une mousse. Les mousses issues des deux compositions (B') et (C'), non conformes à l'invention, ont été testés ensuite sur les cheveux.
On a prélevé l'équivalent d'une mandarine de mousse que l'on a étalée rapidement sur les mains puis on a appliqué la mousse à l'aide des mains sur les cheveux lavés et séchés. Les deux mousses mouillent les cheveux, la mise en forme de la chevelure nécessite un brushing. Le volume de coiffure est faible.

### EXEMPLE 2

On a préparé une mousse de coiffage de composition suivante :
- Terpolymère acide acrylique / acrylate d'éthyle N-tertio butylacrylamide vendu sous la dénomination ULTRAHOLD STRONG par la société BASF (polymère fixant) g 8 g
- Amino-2 méthyl-2 propanol-1 (qs neutralisation à 100% du polymère fixant) 0,8 g
- Ethanol 49,5 g
- Silicone oxyalkylénée vendue sous la dénomination SILICONE FLUID FZ-2172 par la société OSI 2,14 gMA
- Eau qsp 100 g

### Conditionnement en aérosol:

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N" par la société ELF AQUITAINE.

### EXEMPLE 3

On a préparé une mousse de coiffage de composition suivante :
- Terpolymère acide acrylique / acrylate d'éthyle N-tertio butylacrylamide vendu sous la dénomination ULTRAHOLD STRONG par la société BASF(polymère fixant) 8 g
- Amino-2 méthyl-2 propanol-1 (qs neutralisation à 100% du polymère fixant) 0,8 g
- Ethanol 49,5 g
- Silicone oxyalkylénée vendue sous la dénomination SILICONE FLUID FZ-2172 par la société OSI 0,07 gMA
- Eau qsp 100 g

### Conditionnement en aérosol:

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N" par la société ELF AQUITAINE.

### EXEMPLE 4

On a préparé une mousse de coiffage de composition suivante :
- Terpolymère acide acrylique / acrylate d'éthyle N-tertio butylacrylamide vendu sous la dénomination ULTRAHOLD STRONG par la société BASF(polymère fixant) 8 g
- Amino-2 méthyl-2 propanol-1 (qs neutralisation à 100% du polymère fixant) 0,8 g
- Ethanol 43 g
- Silicone oxyéthylénée et oxypropylénée de formule (II) vendue par la société DOW CORNING sous la dénomination FLUID DC 190 4 g
- Eau qsp 100 g

### Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N" par la société ELF AQUITAINE.

### EXEMPLE 5

On a préparé une mousse de coiffage de composition suivante :
- Terpolymère acétate de vinyle /p-tertiobutyl benzoate de vinyle/ acide crotonique vendu sous la dénomination MEXOMERE PW par la société CHIMEX(polymère fixant) 8 g
- Amino-2 méthyl-2 propanol-1 (qs neutralisation à 100% du polymère fixant) 0,8 g
- Ethanol 49,5 g
- Silicone oxyéthylénée de formule (II) vendue par la société GOLDSCHMIDT sous la dénomination ABIL B 8842 2 g
- Eau qsp 100 g

### Conditionnement en aérosol :

90g de la composition ci-dessus sont conditionnés dans un récipient aérosol en présence de 10g d'un mélange ternaire de n-butane, isobutane et propane, (23/55/22), vendu sous la dénomination de "AEROGAZ 3,2 N" par la société ELF AQUITAINE.

Les compositions des exemples 2 à 5 ci-dessus ont donné des résultats équivalents à ceux obtenus avec la composition selon l'invention de l'exemple 1.

## Revendications

1. Composition cosmétique à l'état pressurisée dans un dispositif aérosol en présence d'un agent propulseur et formant une mousse à la sortie dudit dispositif, **caractérisée par le fait qu'**elle comprend, dans un milieu cosmétiquement acceptable, au moins un polymère fixant, au moins une silicone oxyalkylénée comportant au moins un groupe oxyalkyléné de type (-CₓH₂ₓO)ₐ dans lequel x varie de 2 à 6 et a est supérieur ou égal à 1, et au moins 30% en poids, exprimé par rapport à la composition exempte d'agent propulseur, d'un solvant hydrosoluble à plus de 5% et ayant un point d'ébullition inférieur à 85°C, comprenant de l'eau dans des concentrations comprises entre 20 et 65% en poids d'eau par rapport au poids total de la composition, système propulseur non compris.

2. Composition selon la revendication 1, **caractérisée par le fait que** les silicones oxyalkylénées sont choisies parmi les composés de formules générales (VII), (VIII), (IX) et (X): 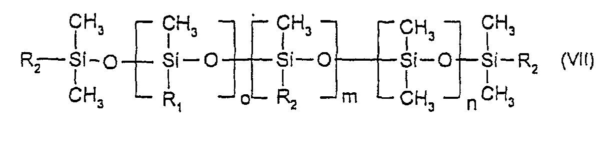 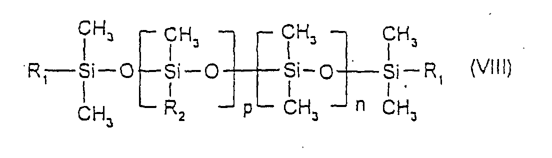 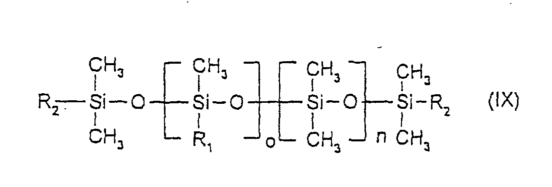 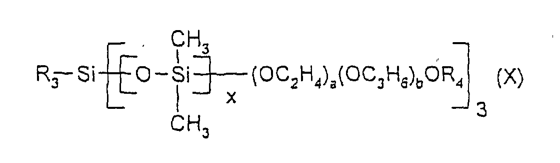 formules dans lesquelles :
- R₁, identique ou différent, représente un radical alkyle, linéaire ou ramifié, en C₁-C₃₀ ou phényle.
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C₁-C₁₂, et de préférence le radical méthyle,
- R₅, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 12 atomes de carbone, un radical hydroxyle, -SO₃M, -OCOR₆, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO(CH₂)_{d}COOM, -OCOCHR₇(CH₂)_{d}COOM, -NHCO(CH₂)_{d}OH, -NH₃Y,
- M, identique ou différent, désigne un atome d'hydrogène, Na, K, Li, NH₄ ou une amine organique,
- R₆ désigné un radical alkyle, linéaire ou ramifié, en C₁-C₃₀,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 1,
- c varie de 0 à 4,
- x varie de 1 à 100,
- Y représente un anion minéral ou organique monovalent.

3. Composition selon la revendication 2, **caractérisée par le fait que** les silicones oxyalkylénées répondent à la formule générale (VII) ou (VIII).

4. Composition selon la revendication 3, **caractérisée par le fait que** les silicones oxyalkylénées répondent à la formule générale (VII) ou (VIII) et répondent à au moins une et de préférence toutes les conditions suivantes :
- c est égal 2 ou 3.
- R₁ désigne le radical méthyle.
- R₅ représente un atome d'hydrogène, un radical méthyle ou un radical acétyle et de préférence un atome d'hydrogène.
- a varie de 1 à 25 et plus particulièrement de 2 à 15.
- b est égal à 0.
- n varie de 0 à 100.
- p varie de 1 à 20.

5. Composition selon la revendication 1, **caractérisée en ce que** la silicone oxyalkylénée présente la formule (XI):
([Y(R₂SiO)ₐ R'₂SiYO][(CₙH₂ₙO)_{b}])_{c} (XI)
formule dans laquelle :
- R et R', identiques ou différents, représentent un radical hydrocarboné monovalent,
- n est un nombre entier allant de 2 à 4,
- a est un nombre supérieur ou égal à 4, de préférence compris entre 4 et 200 et encore plus particulièrement entre 4 et 100.
- b est un nombre supérieur ou égal à 4, de préférence compris entre 4 et 200 et encore plus particulièrement entre 5 et 100.
- c est un nombre supérieur ou égal à 4, de préférence compris entre 4 et 1000 et encore plus particulièrement entre 5 et 300.
- Y représente un groupe organique divalent qui est lié à l'atome de silicium adjacent par une liaison carbone-silicium et à un bloc polyoxyalkylène par un atome d'oxygène,

6. Composition selon la revendication 5, carractérisée en ce que R et R' sont choisis parmi le groupe comprenant les radicaux alkyls linéaires ou ramifiés comme par exemple les radicaux méthyle, éthyle, propyle, butyle, pentyle, hexyle, octyle, décyle, dodécyle, les radicaux aryls comme par exemple phényle, naphtyle, les radicaux aralkyls comme par exemple benzyle, phényléthyle, les radicaux tolyle, xylyle,
Y est-R"-, -R"-CO-, -R"-NHCO-, -R"-NH-CO-NH-R'''-NHCO, -R"-OCONH-R"'-NHCO-, où R" est un groupe alkylène divalent linéaire ou ramifié en C₁-C₆ et R"' est un groupe alkylène divalent ou un groupe arylène divalent comme -C₆H₄-,-C₆H₄-C₆H₄-, -C₆H₄-CH₂-C₆H₄-, -C₆H₄-C(CH₃)₂-C₆H₄-.

7. Composition selon l'une quelconque des revendications 5 ou 6, **caractérisée en ce que** la silicone oxyalkylénée répond à la formule (XII) :
-[C₄H₈ O(C₂H₄O)_{b} (C₃H₆O)_{d} - C₄H₈ - (SiMe₂O)ₐSiMe₂]_{c} (XII)
dans laquelle :
- Me représente méthyle,
- a est un nombre compris entre 4 et 100,
- b et d, identiques ou différents sont des nombres allant de 0 à 100,
- c est un nombre allant de 5 à 300,
- b + d est un nombre allant de 1 à 200.

8. Composition selon l'une quelconque des revendications précédentes, **caractérisée en ce que** le polymère fixant est choisi parmi les polymères anioniques, cationiques, amphotères, non ioniques et leurs mélanges.

9. Composition selon la revendication 8, **caractérisée par le fait que** le polymère fixant anionique est choisi parmi :
- les polymères comportant des motifs carboxyliques dérivant de monomères mono ou diacides carboxyliques insaturés de formule : 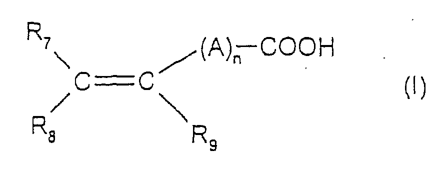 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesuifonique, acrylamido alkylsulfonique.

10. Composition selon la revendication 9, **caractérisée en ce que** le polymère fixant anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères d'acide acrylique ou méthacryliques avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ ;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ;
D) les copolymères dérivés d'acides ou d'anhydrides carboxyliques monoinsaturés en C4-C8 choisis parmi :
- les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées.;
- les copolymères comprenant (i) un ou plusieurs anhydrides maléique, citraconique, itaconique et (II) un ou plusieurs monomères choisis parmi les esters allyliques ou méthallyliques comportant éventuellement un ou plusieurs groupement acrylamide, méthacrylamide, a-oléfine, esters acryliques ou méthacryliques, acides acrylique ou méthacrylique-ou vinylpyrrolidone dans leur chaîne,
les fonctions anhydrides de ces copolymères étant éventuellement monoestérifiées ou monoamidifiées.
E) les polyacrylamides comportant des groupements carboxylates.

11. Composition selon la revendication 10, **caractérisée en ce que** le polymère fixant anionique est choisi parmi :
- les copolymères d'acide acrylique tels que les terpolymères acide acrylique/acrylate d'éthyle/N-tertiobutylacrylamide ;
- les copolymères dérivés d'acide crotonique tels que les terpolymères acétate de vinyle /tertio-butyl benzoate de vinyle/acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- les copolymères d'acide méthacrylique et d'acrylate d'éthyle;
- les copolymères acétate de vinyle/acide crotonique ;
- les terpolymères acétate de vinyle/acide crotonique/polyéthylèneglycol.

12. Composition selon la revendication 8, **caractérisée en ce que** le polymère fixant amphotère est choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

13. Composition selon la revendication 12, **caractérisée en ce que** le polymère fixant amphotère est choisi parmi les copolymères dont la dénomination CTFA est Octylacrylamide/ acrylates/ butylaminoethylmethacrylate copolymer et les copolymères méthacrylate de méthyle / diméthyl carboxyméthylammonio éthylméthacrylate de méthyle.

14. Composition selon la revendication 8, **caractérisé par le fait que** le polymère fixant non Ionique est choisi parmi :
- les polyalkyloxazoline ;
- les homopolymères d'acétate de vinyle;
- les copolymères d'acétate de vinyle et d'ester acrylique ;
- les copolymères d'acétate de vinyle et d'éthylène ;
- les homopolyméres de chlorure de vinyle ;
- les cires de polyéthylène ;
- les cires de polyéthylène/polytétrafluoroéthylène ;
- les copolymères de polyéthylène et d'anhydride maléïque ;
- les homopolymères d'acrylates d'alkyle et les homopolymères de méthacrylates d'alkyle ;
- les copolymères d'esters acryliques tels que par exemple les copolymères d'acrylates d'alkyle et de méthacrylates d'alkyles ;
- les copolymères d'acrylonitrile et d'un monomère non ionique choisi par exemple parmi le butadiène et les (méth)acrylates d'alkyle ;
- les homopolymères de styrène ;
- les copolymères de styrène et de (méth)acrylate d'alkyle ;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne.

15. Composition selon la revendication 8, **caractérisée en ce que** le polymère fixant cationique est choisi parmi :
- les copolymères d'acrylamide et de diméthylaminoéthyl méthacrylate quaternisé au sulfate de diméthyle,
- les copolymères d'acrylamide et de chlorure de méthacryloyloxyéthyltriméthylammonium,
- les copolymères d'acrylamide et de méthosulfate de méthacryloyloxyéthyltriméthylammonium,
- les copolymères vinylpyrrolidone / acrylate ou méthacrylate de dialkylaminoalkyle quaternisés ou non,
- les terpolymères méthacrylate de diméthyl amino éthyle/ vinylcaprolactame/ vinylpyrrolidone,
- et les copolymères vinylpyrrolidone / méthacrylamide de diméthylaminopropyle quaternisé.

16. Composition selon l'une quelconque des revendications précédentes, **caractérisée par le fait que** le solvant hydrosoluble ayant un point d'ébullition inférieur à 85°C est choisi parmi les alcools en C₂-C₆.

17. Composition selon la revendication 16, **caractérisée par le fait que** ledit solvant hydrosoluble est choisi parmi l'éthanol, le tertiobutanol et l'isopropanol.

18. Composition selon l'une quelconque des revendications précédentes, **caractérisée par le fait que** le ou les agents propulseurs sont choisis parmi les hydrocarbures volatils tels que le n-butane, le propane, l'isobutane, les hydrocarbures chlorés et/ou fluorés, le gaz carbonique, le protoxyde d'azote, le diméthyléther, l'azote, l'air comprimé et leur mélange.

19. Composition selon l'une quelconque des revendications précédentes, **caractérisée par le fait que** le ou les polymères fixants sont présents dans des proportions comprises entre 5 % et 40 % en poids environ par rapport au poids total de la composition sans prise en compte de l'agent propulseur, et de préférence entre 6 et 15 %.

20. Composition selon l'une quelconque des revendications précédentes, **caractérisée par le fait que** la ou les silicones oxyalkylénées sont présentes dans des proportions comprises entre 0,01 et 10 % en poids environ par rapport au poids total de la composition sans prise en compte de l'agent propulseur, et de préférence entre 0,05 et 5 %.

21. Composition selon l'une quelconque des revendications précédentes, **caractérisée par le fait que** la ou les solvants hydrosolubles ayant un point d'ébullition inférieur à 85°C sont présents dans des concentrations comprises entre 30 et 60%, ces concentrations étant exprimées en poids par rapport au poids total de la composition sans prise en compte de l'agent propulseur.

22. Composition selon l'une quelconque des revendications précédentes, **caractérisée par le fait que** le ou les agents propulseurs sont présents dans des concentrations comprises entre 1 et 20% en poids par rapport au poids total de la composition à l'état pressurisée, et de préférence entre 5 et 15% en poids.

23. Composition selon l'une quelconque des revendications précédentes, **caractérisée par le fait qu'**elle contient également des agents tensio-actifs, des agents épaississants, des agents conservateurs, des séquestrants, des adoucissants, des colorants, des agents modificateurs de viscosité, des agents modificateurs de mousse, des stabilisateurs de mousse, des agents nacrants, des agents hydratants, des agents antipelliculaires, des agents antiséborrhéiques, des filtres solaires, des protéines, des vitamines, des plastifiants, des hydroxyacides, des électrolytes, des parfums, et des agents conditionneurs.

24. Composition cosmétique sous forme de mousse, **caractérisée par le fait qu'**elle résulte de l'expansion à l'air d'une composition telle que définie à l'une quelconques des revendications 1 à 23.

25. Utilisation d'une silicone oxyalkylénée comportant au moins un groupe oxyalkyléné de type (-CₓH₂ₓO)ₐ dans lequel x varie de 2 à 6 et a est supérieur ou égal à 1 en tant qu'agent de moussage des compositions pressurisées dans un dispositif aérosol susceptibles de former une mousse à la sortie dudit dispositif et comprenant au moins un polymère fixant, au moins 30% en poids d'un solvant hydrosoluble à plus de 5%, ayant un point d'ébullition inférieur à 85°C, cette conconcentration étant exprimée par rapport à la composition exempte d'agent propulseur.

26. Procédé de traitement cosmétique des fibres kératiniques, **caractérisé par le fait que** l'on applique sur des fibres kératiniques une composition telle que définie à l'une quelconque des revendications 1 à 24.

## Patentansprüche

1. Kosmetische Zusammensetzung, die in Gegenwart eines Treibmittels in einer Aerosolvorrichtung unter Druck steht und am Auslaß der Vorrichtung einen Schaum bildet, **dadurch gekennzeichnet, daß** sie in einem kosmetisch akzeptablen Medium mindestens ein fixierendes Polymer, mindestens ein alkoxyliertes Silicon, das mindestens eine alkoxylierte Gruppe vom Typ (-CₓH₂ₓO)ₐ aufweist, wobei x im Bereich von 2 bis 6 liegt und a mindestens 1 ist, und mindestens 30 Gew.-% eines bei mehr als 5 % wasserlöslichen Lösungsmittels mit einem Siedepunkt unter 85 °C, bezogen auf die Zusammensetzung ohne Treibmittel, enthält, wobei sie Wasser in Konzentrationen von 20 bis 65 Gew.-%, bezogen auf das Gesamtgewicht der Zusammensetzung ohne Berücksichtigung des Treibmittelsystems, enthält.

2. Zusammensetzung nach Anspruch 1, **dadurch gekennzeichnet, daß** die alkoxylierten Silicone unter den Verbindungen der allgemeinen Formeln (VII), (VIII), (IX) und (X) ausgewählt sind: 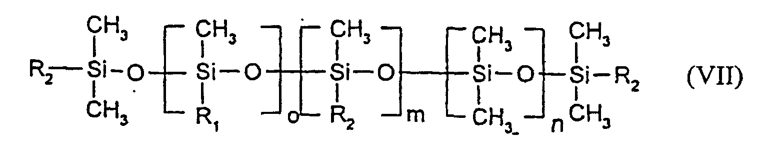 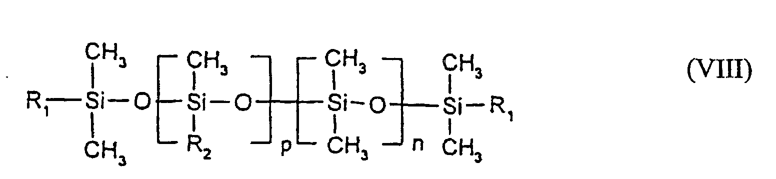 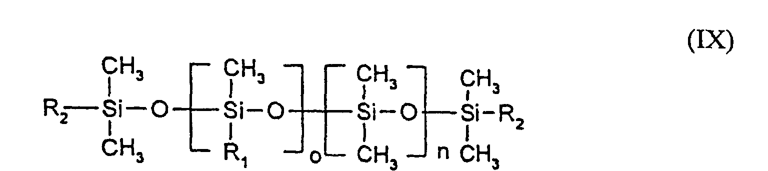 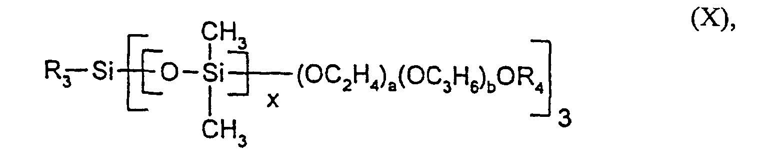 wobei in den Formeln
- die Gruppen R₁, die identisch oder voneinander verschieden sind, eine geradkettige oder verzweigte C₁₋₃₀-Alkylgruppe oder Phenyl bedeuten,
- die Gruppen R₂, die identisch oder voneinander verschieden sind, eine Gruppe -C_{c}H_{2c}-O-(C₂H₄O)ₐ(C₃H₆O)_{b}-R₅ oder eine Gruppe -C_{c}H_{2c}-O-(C₄H₈O)ₐ-R₅ bedeuten,
- die Gruppen R₃ und R₄, die identisch oder voneinander verschieden sind, eine geradkettige oder verzweigte C₁₋₁₂-Alkylgruppe und vorzugsweise Methyl bedeuten,
- die Gruppen R₅, die identisch oder voneinander verschieden sind, unter Wasserstoff, geradkettigen oder verzweigten Alkylgruppen mit 1 bis 12 Kohlenstoffatomen, geradkettigen oder verzweigten Alkoxygruppen mit 1 bis 6 Kohlenstoffatomen, geradkettigen oder verzweigten Acylgruppen mit 2 bis 12 Kohlenstoffatomen, Hydroxy, -SO₃M, -OCOR₆, C₁₋₆-Aminoalkoxygruppen, die gegebenenfalls an der Aminogruppe substituiert sind, C₂₋₆-Aminoacylgruppen, die gegebenenfalls an der Aminogruppe substituiert sind, -NHCH₂CH₂COOM, -N(CH₂CH₂COOM)₂, Aminoalkylgruppen, die gegebenenfalls an der Aminogruppe und der Alkylgruppe substituiert sind, C₂₋₃₀-Carboxyacyl, Phosphonogruppen, die gegebenenfalls mit einer oder zwei substituierten Aminoalkylgruppen substituiert sind, -CO(CH₂)_{d}COOM, -OCOCHR₇(CH₂)_{d}COOM, -NHCO(CH₂)_{d}OH oder -NH₃Y ausgewählt sind,
- die Gruppen M, die identisch oder voneinander verschieden sind, Wasserstoff, Na, K, Li, NH₄ oder ein organisches Amin bedeuten,
- R₅ eine geradkettige oder verzweigte C₁₋₃₀-Alkylgruppe bedeutet,
- R₇ Wasserstoff oder SO₃M bedeutet,
- d im Bereich von 1 bis 10 liegt,
- m im Bereich von 0 bis 20 liegt,
- n im Bereich von 0 bis 500 liegt,
- o im Bereich von 0 bis 20 liegt,
- p im Bereich von 1 bis 50 liegt,
- a im Bereich von 0 bis 50 liegt,
- b im Bereich von 0 bis 50 liegt,
- a + b mindestens 1 ist,
- c im Bereich von 0 bis 4 liegt,
- x im Bereich von 1 bis 100 liegt und
- Y ein einwertiges anorganisches oder organisches Anion bedeutet.

3. Zusammensetzung nach Anspruch 2, **dadurch gekennzeichnet, daß** die alkoxylierten Silicone der allgemeinen Formel (VII) oder (VIII) entsprechen.

4. Zusammensetzung nach Anspruch 3, **dadurch gekennzeichnet, daß** die alkoxylierten Silicone der allgemeinen Formel (VII) oder (VIII) entsprechen und mindestens eine und vorzugsweise alle folgenden Bedingungen erfüllen:
- c ist 2 oder 3,
- R₁ bedeutet Methyl,
- R₅ bedeutet Wasserstoff, Methyl oder Acetyl und vorzugsweise Wasserstoff,
- a liegt im Bereich von 1 bis 25 und insbesondere 2 bis 15,
- b ist 0,
- n liegt im Bereich von 0 bis 100, und
- p liegt im Bereich von 1 bis 20.

5. Zusammensetzung nach Anspruch 1, **dadurch gekennzeichnet, daß** das alkoxylierte Silicon die Formel (XI) aufweist:
([Y(R₂SiO)ₐR'₂SiYO][(CₙH₂ₙO)_{b}])_{c} (XI)
worin bedeuten:
- die Gruppen R und R', die identisch oder voneinander verschieden, sind eine einwertige Kohlenwasserstoffgruppe,
- n eine ganze Zahl von 2 bis 4,
- a mindestens 4 und vorzugsweise eine Zahl im Bereich von 4 bis 200 und insbesondere im Bereich von 4 bis 100,
- b mindestens 4 und vorzugsweise eine Zahl im Bereich von 4 bis 200 und insbesondere 5 bis 100,
- c mindestens 4 und vorzugsweise eine Zahl im Bereich von 4 bis 1000 und insbesondere 5 bis 300,
- Y eine zweiwertige organische Gruppe, die über eine Kohlenstoff-Silicium-Bindung an das angrenzende Siliciumatom und über ein Sauerstoffatom an eine Polyoxyalkylengruppe gebunden ist.

6. Zusammensetzung nach Anspruch 5, **dadurch gekennzeichnet, daß** die Gruppen R und R' unter den geradkettigen oder verzweigten Alkylgruppen, wie beispielsweise Methyl, Ethyl, Propyl, Butyl, Pentyl, Hexyl, Octyl, Decyl und Dodecyl, den Arylgruppen, wie beispielsweise Phenyl und Naphthyl, und den Aralkylgruppen, wie beispielsweise Benzyl, Phenylethyl, Tolyl und Xylyl, ausgewählt sind,
Y -R"-, -R"-CO-, -R"-NHCO-, -R"NH-CO-NH-R'''-NHCO oder -R"-OCONH-R"'-NHCO- bedeutet, wobei R" eine geradkettige oder verzweigte C₁₋₆-Alkylengruppe ist und R''' eine zweiwertige Alkylengruppe oder eine zweiwertige Arylengruppe bedeutet, wie -C₆H₄-, -C₆H₄-C₆H₄-, -C₆H₄-CH₂-C₆H₄- und -C₆H₄C(CH₃)₂-C₆H₄-.

7. Zusammensetzung nach einem der Ansprüche 5 oder 6, **dadurch gekennzeichnet, daß** das alkoxylierte Silicon der Formel (XII) entspricht:
[C₄H₈O(C₂H₄O)_{b}(C₃H₆O)_{d} - C₄H₈ - (SiMe₂O)ₐSiMe₂]_{c} (XII)
worin bedeuten:
- Me Methyl,
- a eine Zahl im Bereich von 4 bis 100,
- b und d, die identisch oder voneinander verschieden, Zahlen im Bereich von 0 bis 100,
- c eine Zahl von 5 bis 300, und
- b + c eine Zahl im Bereich von 1 bis 200.

8. Zusammensetzung nach einem der vorhergehenden Ansprüche, **dadurch gekennzeichnet, daß** das fixierende Polymer unter den anionischen, kationischen, amphoteren oder nichtionischen Polymeren und deren Gemischen ausgewählt ist.

9. Zusammensetzung nach Anspruch 8, **dadurch gekennzeichnet, daß** das anionische fixierende Polymer ausgewählt ist unter:
- den Polymeren, die Carboxyeinheiten aufweisen, die von ungesättigten Mono- oder Dicarbonsäuremonomeren der Formel: 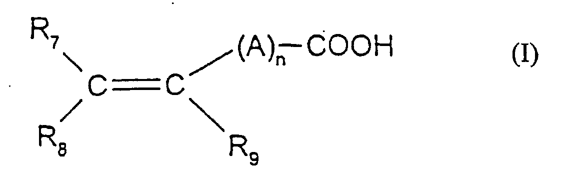 abgeleitet sind, wobei n 0 oder eine ganze Zahl von 1 bis 10 bedeutet, A eine Methylengruppe ist, die gegebenenfalls an das Kohlenstoffatom der ungesättigten Gruppe oder, wenn n größer 1 ist, an die benachbarte Methylengruppe über ein Heteroatom, wie Sauerstoff oder Schwefel, gebunden ist, R₇ Wasserstoff, Phenyl oder Benzyl bedeutet, R₈ Wasserstoff, eine niedere Alkylgruppe oder Carboxy bedeutet, und R₉ Wasserstoff, eine niedere Alkylgruppe, -CH₂-COOH, Phenyl oder Benzyl bedeutet; und
- den Polymeren, die Einheiten enthalten, die von Sulfonsäuren abgeleitet sind, wie Vinylsulfonsäureeinheiten, Styrolsulfonsäureeinheiten und Acrylamidoalkylsulfonsäureeinheiten.

10. Zusammensetzung nach Anspruch 9, **dadurch gekennzeichnet, daß** das anionische fixierende Polymer ausgewählt ist unter:
A) den Homo- oder Copolymeren von Acrylsäure oder Methacrylsäure oder deren Salzen, Copolymeren von Acrylsäure und Acrylamid und deren Salzen und Natriumsalzen von Polyhydroxycarbonsäuren;
B) den Copolymeren von Acrylsäure oder Methacrylsäure und einem monoethylenisch ungesättigten Monomer, wie Ethylen, Styrol, Vinylestern, Acrylsäure- oder Methacrylsäureestern, die gegebenenfalls mit einem Polyalkylenglykol, wie Polyethylenglykol, gepfropft sind und gegebenenfalls vernetzt sind; den Copolymeren dieses Typs, die in der Kette eine gegebenenfalls N-alkylierte und/oder hydroxyalkylierte Acrylamideinheit enthalten, und den Copolymeren von Acrylsäure und C₁₋₄-Alkylmethacrylat;
C) den Copolymeren, die von Crotonsäure abgeleitet sind, wie den Copolymeren, die in ihrer Kette Vinylacetat- oder Vinylpropionateinheiten und gegebenenfalls weitere Monomere enthalten, wie Allyl- oder Methallylester, Vinylether oder Vinylester einer gesättigten, geradkettigen oder verzweigten Carbonsäure mit langer Kohlenwasserstoffkette, beispielsweise Verbindungen, die mindestens 5 Kohlenstoffatome enthalten, wobei diese Polymere gegebenenfalls gepfropft und vemetzt vorliegen können;
D) den Copolymeren, die von einfach ungesättigten Carbonsäuren oder Carbonsäureanhydriden mit 4 bis 8 Kohlenstoffatomen abgeleitet sind, die ausgewählt sind unter:
- den Copolymeren, die (i) Maleinsäure, Fumarsäure, Itaconsäure oder deren Anhydride oder deren Gemische und (ii) mindestens ein Monomer enthalten, das unter den Vinylestern, Vinylethern, Vinylhalogeniden, Phenylvinylderivaten und Acrylsäure und ihren Estern ausgewählt ist, wobei die Anhydridfunktionen dieser Polymere gegebenenfalls einfach verestert oder einfach amidiert sind; und
- den Copolymeren, die (i) Maleinsäure, Citraconsäure, Itaconsäure oder deren Anhydride oder deren Gemische und (ii) ein oder mehrere Monomere enthalten, die unter den Allylestern oder Methallylestern ausgewählt sind, die gegebenenfalls eine oder mehrere Gruppen Acrylamid, Methacrylamid, α-Olefin, Acrylester oder Methacrylester, Acrylsäure oder Methacrylsäure oder Vinylpyrrolidon in ihrer Kette enthalten,
wobei die Anhydridfunktionen dieser Copolymere gegebenenfalls einfach verestert oder amidiert sind; und
(E) den Polyacrylamiden, die Carboxygruppen aufweisen.

11. Zusammensetzung nach Anspruch 10, **dadurch gekennzeichnet, daß** das anionische fixierende Polymer ausgewählt ist unter:
- den Acrylsäurecopolymeren, beispielsweise den Acrylsäure/Ethylacrylat/N-*tert*.-Butylacrylamid-Terpolymeren;
- den Copolymeren, die von Crotonsäure abgeleitet sind, beispielsweise den Vinylacetat/Vinyl-*tert*.-Butylbenzoat/Crotonsäure-Terpolymeren und den Crotonsäure/Vinylacetat/Vinylneododecanoat-Terpolymeren;
- den Polymeren, die von Maleinsäure, Fumarsäure, Itaconsäure oder deren Anhydriden mit Vinylestern, Vinylethern, Vinylhalogeniden, Phenylvinylderivaten und Acrylsäure und ihren Estern abgeleitet sind, beispielsweise den Copolymeren von Methylvinylether und einfach verestertem Maleinsäureanhydrid;
- den Copolymeren von Methacrylsäure und Methylmethacrylat;
- den Copolymeren von Methacrylsäure und Ethylacrylat;
- den Copolymeren von Vinylacetat und Crotonsäure; und
- den Vinylacetat/Crotonsäure/Polyethylenglykol-Terpolymeren.

12. Zusammensetzung nach Anspruch 8, **dadurch gekennzeichnet, daß** das amphotere fixierende Polymer unter den Polymeren ausgewählt ist, die Einheiten aufweisen, welche abgeleitet sind von:
a) mindestens einem Monomer, das unter den Acrylamiden oder Methacrylamiden ausgewählt ist, die am Stickstoffatom mit einer Alkylgruppe substituiert sind,
b) mindestens einem Säurecomonomer, das eine oder mehrere reaktive Carboxygruppen aufweist, und
c) mindestens einem basischen Comonomer, beispielsweise Acrylsäureestern und Methacrylsäureestern mit primären, sekundären oder tertiären Aminosubstituenten und quartären Ammoniumsubstituenten, und dem Quaternisierungsprodukt von Dimethylaminoethylmethacrylat und Dimethylsulfat oder Diethylsulfat.

13. Zusammensetzung nach Anspruch 12, **dadurch gekennzeichnet, daß** das amphotere fixierende Polymer unter den Copolymeren, die nach CTFA-Nomenklatur als Octylacrylamid/Acrylate/Butylaminoethylmethacrylat-Copolymere bezeichnet werden, und den Methylmethacrylat/Dimethylcarboxymethylammoniumethylmethacrylsäuremethylester-Copolymeren ausgewählt ist.

14. Zusammensetzung nach Anspruch 8, **dadurch gekennzeichnet, daß** das nichtionische fixierende Polymer ausgewählt ist unter:
- den Polyalkyloxazolinen;
- den Homopolymeren von Vinylacetat;
- den Copolymeren von Vinylacetat und Acrylestern;
- den Copolymeren von Vinylacetat und Ethylen;
- den Homopolymeren von Vinylchlorid;
- den Polyethylenwachsen;
- den Polyethylen/Polytetrafluorethylen-Wachsen;
- den Copolymeren von Polyethylen und Maleinsäureanhydrid;
- den Homopolymeren von Alkylacrylaten und den Homopolymeren von Alkylmethacrylaten;
- den Acrylester-Copolymeren, wie beispielsweise den Copolymeren von Alkylacrylaten und Alkylmethacrylaten;
- den Copolymeren von Acrylnitril und einem nichtionischen Monomer, das beispielsweise unter Butadien und den Alkyl(meth)acrylaten ausgewählt ist;
- den Styrol-Homopolymeren;
- den Copolymeren von Styrol und Alkyl(meth)acrylat;
- den Copolymeren von Styrol, Alkyl(meth)acrylat und Alkylacrylat;
- den Copolymeren von Styrol und Butadien;
- den Copolymeren von Styrol, Butadien und Vinylpyridin; und
- den Copolymeren von Alkylacrylat und Urethan.

15. Zusammensetzung nach Anspruch 8, **dadurch gekennzeichnet, daß** das kationische fixierende Polymer ausgewählt ist unter:
- den Copolymeren von Acrylamid und mit Dimethylsulfat quaternisiertem Dimethylaminoethylmethacrylat,
- den Copolymeren von Acrylamid und Methacryloyloxyethyltrimethylammoniumchlorid,
- den Copolymeren von Acrylamid und Methacryloyloxyethyltrimethylammoniummethosulfat,
- den Copolymeren von Vinylpyrrolidon/Dialkylaminoalkylacrylat oder -methacrylat, die gegebenenfalls quaternisiert sind,
- den Dimethylaminoethylmethacrylat/Vinylcaprolactam/Vinylpyrrolidon-Terpolymeren, und
- den Copolymeren von Vinylpyrrolidon und quaternisiertem Dimethylaminopropylmethacrylamid.

16. Zusammensetzung nach einem der vorhergehenden Ansprüche, **dadurch gekennzeichnet, daß** das wasserlösliche Lösungsmittel mit einem Siedepunkt unter 85 °C unter den C₂₋₆-Alkoholen ausgewählt ist.

17. Zusammensetzung nach Anspruch 16, **dadurch gekennzeichnet, daß** das wasserlösliche Lösungsmittel unter Ethanol, tert.-Butanol und Isopropanol ausgewählt ist.

18. Zusammensetzung nach einem der vorhergehenden Ansprüche, **dadurch gekennzeichnet, daß** das Treibmittel oder die Treibmittel unter den flüchtigen Kohlenwasserstoffen, wie n-Butan, Propan, Isobutan, chlorierten und/oder fluorierten Kohlenwasserstoffen, Kohlendioxid, Distickstoffoxid, Dimethylether, Stickstoff, Druckluft und deren Gemischen ausgewählt ist.

19. Zusammensetzung nach einem der vorhergehenden Ansprüche, **dadurch gekennzeichnet, daß** das oder die fixierenden Polymere in Mengenanteilen von etwa 5 bis 40 Gew.-%, bezogen auf das Gesamtgewicht der Zusammensetzung, wobei das Treibmittel nicht eingeschlossen ist, und vorzugsweise im Bereich von 6 bis 15 % vorliegen.

20. Zusammensetzung nach einem der vorhergehenden Ansprüche, **dadurch gekennzeichnet, daß** das alkoxylierte Silicon oder die alkoxylierten Silicone in Mengenanteilen von etwa 0,01 bis 10 Gew.-%, bezogen auf das Gesamtgewicht der Zusammensetzung, wobei das Treibmittel nicht eingeschlossen ist, und vorzugsweise im Bereich von 0,05 bis 5 % enthalten sind.

21. Zusammensetzung nach einem der vorhergehenden Ansprüche, **dadurch gekennzeichnet, daß** das oder die wasserlöslichen Lösungsmittel mit einem Siedepunkt unter 85 °C in Konzentrationen von 30 bis 60 % vorliegen, wobei die Konzentrationen in Gewichtsprozent, bezogen auf das Gesamtgewicht der Zusammensetzung ohne Berücksichtigung des Treibmittels, ausgedrückt sind.

22. Zusammensetzung nach einem der vorhergehenden Ansprüche, **dadurch gekennzeichnet, daß** das oder die Treibmittel in Konzentrationen von 1 bis 20 Gew.-%, bezogen auf das Gesamtgewicht der unter Druck stehenden Zusammensetzung, und vorzugsweise im Bereich von 5 bis 15 Gew.-% vorliegen.

23. Zusammensetzung nach einem der vorhergehenden Ansprüche, **dadurch gekennzeichnet, daß** sie auch grenzflächenaktive Stoffe, Verdickungsmittel, Konservierungsmittel, Maskierungsmittel, Mittel für die Geschmeidigkeit, Färbemittel, Viskositätsregler, Schaummodifikatoren, Schaumstabilisatoren, Perlglanzpigmente, Hydratisierungsmittel, Mittel gegen Schuppen, Mittel gegen Seborrhoe, Sonnenschutzfilter, Proteine, Vitamine, Weichmacher, Hydroxysäuren, Elektrolyte, Parfums und Konditioniermittel enthält.

24. Kosmetische Zusammensetzung, die als Schaum vorliegt, **dadurch gekennzeichnet, daß** sie bei der Expansion einer Zusammensetzung nach einem der Ansprüche 1 bis 23 an Luft entsteht.

25. Verwendung eines alkoxylierten Silicons, das mindestens eine alkoxylierte Gruppe vom Typ (-CₓH₂ₓO)ₐ aufweist, wobei x im Bereich von 2 bis 6 liegt und a mindestens 1 ist, als Schaumbildner in Zusammensetzungen, die in einer Aerosolvorrichtung unter Druck stehen, am Auslaß der Vorrichtung einen Schaum bilden können und mindestens ein fixierendes Polymer und mindestens 30 Gew.-% eines bei mehr als 5 % wasserlöslichen Lösungsmittels mit einem Siedepunkt unter 85 °C enthalten, wobei die Konzentration bezogen auf die Zusammensetzung ohne das Treibmittel ausgedrückt ist.

26. Verfahren zur kosmetischen Behandlung von Keratinfasern, **dadurch gekennzeichnet, daß** auf die Keratinfasern eine Zusammensetzung nach einem der Ansprüche 1 bis 24 aufgetragen wird.

## Claims

1. Cosmetic composition in the pressurized state in an aerosol device in the presence of a propellant which forms a foam at the outlet of the said device, **characterized in that** it comprises, in a cosmetically acceptable medium, at least one fixing polymer, at least one oxyalkylenated silicone comprising at least one oxyalkylenated group of (-CₓH₂ₓO)ₐ type in which x varies from 2 to 6 and a is greater than or equal to 1, and at least 30% by weight, expressed with respect to the propellant-free composition, of a solvent which is soluble in water to more than 5% and which has a boiling point of less than 85°C, and **in that** it comprises water in concentrations of between 20 and 65% by weight of water with respect to the total weight of the composition, the propellant system not included.

2. Composition according to Claim 1, **characterized in that** the oxyalkylenated silicones are chosen from compounds of general formulae .(VII), (VIII), (IX) and (X): 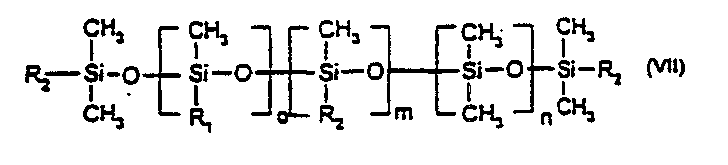 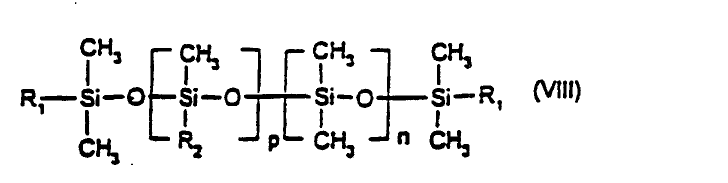 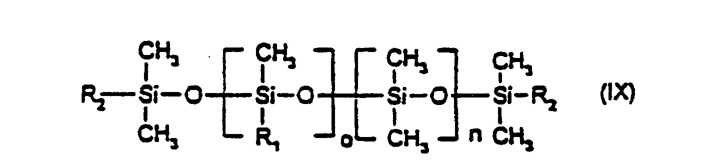 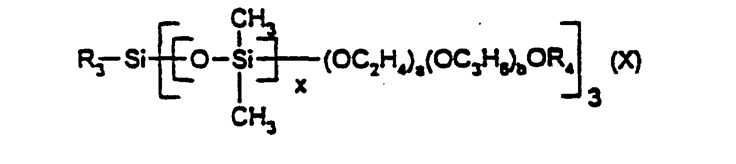 in which formulae:
- R₁, which is identical or different, represents a linear or branched C₁-C₃₀ alkyl or phenyl radical,
- R₂, which is identical or different, represents a -C_{c}H_{2c}-O-(C₂H₄O)ₐ(C₃H₆O)_{b}-R₅ radical or a -C_{c}H_{2c}-O-(C₄H₈O)ₐ-R₅ radical,
- R₃ and R₄, which are identical or different, denote a linear or branched C₁-C₁₂ alkyl radical and preferably the methyl radical,
- R₅, which is identical or different, is chosen from a hydrogen atom, a linear or branched alkyl radical containing 1 to 12 carbon atoms, a linear or branched alkoxy radical containing 1 to 6 carbon atoms, a linear or branched acyl radical containing 2 to 12 carbon atoms, a hydroxyl, -SO₃M, -OCOR₆, C₁-C₆ aminoalkoxy, optionally substituted on the amine, C₂-C₆ aminoacyl, optionally substituted on the amine, -NHCH₂CH₂COOM, -N(CH₂CH₂COOM)₂, aminoalkyl, optionally substituted on the amine and on the alkyl chain, or C₂-C₃₀ carboxyacyl radical or a phosphono, optionally substituted by one or two substituted aminoalkyl radicals, -CO(CH₂)_{d}COOM, -OCOCHR₇(CH₂)_{d}COOM, -NHCO(CH₂)_{d}OH or -NH₃Y group,
- M, which is identical or different, denotes a hydrogen atom, Na, K, Li, NH₄ or an organic amine,
- R₆ denotes a linear or branched C₁-C₃₀ alkyl radical,
- R₇ denotes a hydrogen atom or an SO₃M radical,
- d varies from 1 to 10,
- m varies from 0 to 20,
- n varies from 0 to 500,
- o varies from 0 to 20,
- p varies from 1 to 50,
- a varies from 0 to 50,
- b varies from 0 to 50,
- a + b is greater than or equal to 1,
- c varies from 0 to 4,
- x varies from 1 to 100,
- Y represents a monovalent inorganic or organic anion.

3. Composition according to Claim 2, **characterized in that** the oxyalkylenated silicones correspond to the general formula (VII) or (VIII).

4. Composition according to Claim 3, **characterized in that** the oxyalkylenated silicones correspond to the general formula (VII) or (VIII) and meet at least one and preferably all of the following conditions:
- c is equal to 2 or 3,
- R₁ denotes the methyl radical,
- R₅ represents a hydrogen atom, a methyl radical or an acetyl radical and preferably a hydrogen atom,
- a varies from 1 to 25 and more particularly from 2 to 15,
- b is equal to 0,
- n varies from 0 to 100,
- p varies from 1 to 20.

5. Composition according to Claim 1, **characterized in that** the oxyalkylenated silicone has the formula (XI):
([Y(R₂SiO)ₐR'₂SiYO][(CₙH₂ₙO)_{b}])_{c} (XI)
in which formula
- R and R', which are identical or different, represent a monovalent hydrocarbon radical,
- n is an integer ranging from 2 to 4,
- a is a number greater than or equal to 4, preferably of between 4 and 200 and more particularly still between 4 and 100,
- b is a number greater than or equal to 4, preferably of between 4 and 200 and more particularly still between 5 and 100,
- c is a number greater than or equal to 4, preferably of between 4 and 1000 and more particularly still between 5 and 300,
- Y represents a divalent organic group which is bonded to the adjacent silicon atom via a carbon-silicon bond and to a polyoxyalkylene block via an oxygen atom.

6. Composition according to Claim 5, **characterized in that** R and R' are chosen from the group comprising linear or branched alkyl radicals, such as, for example, the methyl, ethyl, propyl, butyl, pentyl, hexyl, octyl, decyl or dodecyl radicals, aryl radicals, such as, for example, phenyl or naphthyl, or aralkyl radicals, such as, for example, benzyl, phenylethyl or the tolyl or xylyl radicals,
Y is -R"-, -R"-CO-, -R"-NHCO-, -R"-NH-CO-NH-R'''-NHCO- or -R"-OCONH-R'''-NHCO-, where R" is a linear or branched divalent C₁-C₆ alkylene group and R''' is a divalent alkylene group or a divalent arylene group, such as -C₆H₄-, -C₆H₄-C₆H₄-, -C₆H₄-CH₂-C₆H₄- or -C₆H₄-C(CH₃)₂-C₆H₄-.

7. Composition according to either one of Claims 5 and 6, **characterized in that** the oxyalkylenated silicone corresponds to the formula (XII):
[C₄H₈O(C₂H₄O)_{b}(C₃H₆O)_{d}-C₄H₈-(SiMe₂O)ₐSiMe₂]_{c} (XII)
in which:
- Me represents methyl,
- a is a number between 4 and 100,
- b and d, which are identical or different, are numbers ranging from 0 to 100,
- c is a number ranging from 5 to 300,
- b + d is a number ranging from 1 to 200.

8. Composition according to any one of the preceding claims, **characterized in that** the fixing polymer is chosen from anionic, cationic, amphoteric or non-ionic polymers and their mixtures.

9. Composition according to Claim 8, **characterized in that** the anionic fixing polymer is chosen from:
- polymers containing carboxyl units deriving from unsaturated carboxylic mono- or diacid monomers of formula: 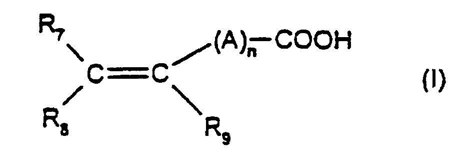 in which n is an integer from 0 to 10, A denotes a methylene group, optionally bonded to the carbon atom of the unsaturated group or to the neighbouring methylene group when n is greater than 1 via a heteroatom, such as oxygen or sulphur, R₇ denotes a hydrogen atom or a phenyl or benzyl group, R₈ denotes a hydrogen atom or a lower alkyl or carboxyl group, and R₉ denotes a hydrogen atom, a lower alkyl group or a -CH₂-COOH, phenyl or benzyl group;
- polymers comprising units deriving from sulphonic acid, such as vinylsulphonic, styrenesulphonic or acrylamidoalkylsulphonic units.

10. Composition according to Claim 9, **characterized in that** the anionic fixing polymer is chosen from:
A) homo- or copolymers of acrylic or methacrylic acid or their salts, copolymers of acrylic acid and of acrylamide and their salts, or the sodium salts of polyhydroxycarboxylic acids;
B) copolymers of acrylic or methacrylic acid with a monoethylenic monomer, such as ethylene, styrene, vinyl esters or esters of acrylic or methacrylic acid, optionally grafted onto a polyalkylene glycol, such as polyethylene glycol, and optionally crosslinked; the copolymers of this type containing, in their chain, an optionally N-alkylated and/or -hydroxyalkylated acrylamide unit; or copolymers of acrylic acid and of C₁-C₄ alkyl methacrylate;
C) copolymers derived from crotonic acid, such as those containing, in their chain, vinyl acetate or propionate units and optionally other monomers, such as allyl or methallyl esters, vinyl ether or vinyl ester of a linear or branched saturated carboxylic acid containing a long hydrocarbon chain, such as those containing at least 5 carbon atoms, it optionally being possible for these polymers to be grafted and crosslinked;
D) copolymers derived from C₄-C₈ monounsaturated carboxylic acids or anhydrides chosen from:
- copolymers comprising (i) one or more maleic, fumaric or itaconic acids or anhydrides and (ii) at least one monomer chosen from vinyl esters, vinyl ethers, vinyl halides, phenylvinyl derivatives, or acrylic acid and its esters, the anhydride functional groups of these copolymers optionally being monoesterified or monoamidated;
- copolymers comprising (i) one or more maleic, citraconic or itaconic anhydrides and (ii) one or more monomers chosen from allyl or methallyl esters, optionally containing one or more acrylamide, methacrylamide, α-olefin, acrylic or methacrylic ester, acrylic or methacrylic acid, or vinylpyrrolidone groups in their chain,
the anhydride functional groups of these copolymers optionally being monoesterified or monoamidated;
E) polyacrylamides containing carboxylate groups.

11. Composition according to Claim 10, **characterized in that** the anionic fixing polymer is chosen from:
- acrylic acid copolymers, such as acrylic acid/ethyl acrylate/N-tert-butylacrylamide terpolymers;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives, or acrylic acid and its esters, such as monoesterified maleic anhydride/methyl vinyl ether copolymers;
- copolymers of methacrylic acid and of methyl methacrylate;
- copolymers of methacrylic acid and of ethyl acrylate;
- vinyl acetate/crotonic acid copolymers;
- vinyl acetate/crotonic acid/polyethylene glycol terpolymers.

12. Composition according to Claim 8, **characterized in that** the amphoteric fixing polymer is chosen from polymers containing units deriving:
a) from at least one monomer chosen from acrylamides or methacrylamides substituted on the nitrogen by an alkyl radical,
b) from at least one acidic comonomer containing one or more reactive carboxyl groups, and
c) from at least one basic comonomer, such as esters containing primary, secondary, tertiary and quaternary amine substituents of acrylic and methacrylic acids and the quaternization product of dimethylaminoethyl methacrylate with dimethyl or diethyl sulphate.

13. Composition according to Claim 12, **characterized in that** the amphoteric fixing polymer is chosen from copolymers with the CTFA name of Octylacrylamide/acrylates/butylaminoethyl methacrylate copolymer and methyl methacrylate/dimethylcarboxy-methylammonioethyl methacrylate copolymers.

14. Composition according to Claim 8, **characterized in that** the non-ionic fixing polymer is chosen from:
- polyalkyloxazolines;
- vinyl acetate homopolymers;
- copolymers of vinyl acetate and of acrylic ester;
- copolymers of vinyl acetate and of ethylene;
- vinyl chloride homopolymers;
- polyethylene waxes;
- polyethylene/polytetrafluoroethylene waxes;
- copolymers of polyethylene and of maleic anhydride;
- alkyl acrylate homopolymers and alkyl methacrylate homopolymers;
- acrylic ester copolymers, such as, for example, copolymers of alkyl acrylates and of alkyl methacrylates;
- copolymers of acrylonitrile and of a non-ionic monomer chosen, for example, from butadiene and alkyl (meth)acrylates;
- styrene homopolymers;
- copolymers of styrene and of alkyl (meth)acrylate;
- copolymers of styrene, of alkyl methacrylate and of alkyl acrylate;
- copolymers of styrene and of butadiene;
- copolymers of styrene, of butadiene and of vinylpyridine;
- copolymers of alkyl acrylate and of urethane.

15. Composition according to Claim 8, **characterized in that** the cationic fixing polymer is chosen from:
- copolymers of acrylamide and of dimethylaminoethyl methacrylate quaternized with dimethyl sulphate,
- copolymers of acrylamide and of methacryloyloxyethyltrimethylammonium chloride,
- copolymers of acrylamide and of methacryloyloxyethyltrimethylammonium methyl sulphate,
- optionally quaternized vinylpyrrolidone/dialkylaminoalkyl acrylate or methacrylate copolymers,
- dimethylaminoethyl methacrylate/vinylcaprolactam/ vinylpyrrolidone terpolymers,
- and quaternized dimethylaminopropylmethacrylamide/ vinylpyrrolidone copolymers.

16. Composition according to any one of the preceding claims, **characterized in that** the water-soluble solvent having a boiling point of less than 85°C is chosen from C₂-C₆ alcohols.

17. Composition according to Claim 16, **characterized in that** the said water-soluble solvent is chosen from ethanol, tert-butanol and isopropanol.

18. Composition according to any one of the preceding claims, **characterized in that** the propellant or propellants are chosen from volatile hydrocarbons, such as n-butane, propane or isobutane, chlorinated and/or fluorinated hydrocarbons, carbon dioxide gas, nitrous oxide, dimethyl ether, nitrogen, compressed air and their mixture.

19. Composition according to any one of the preceding claims, **characterized in that** the fixing polymer or polymers are present in proportions of between 5% and 40% by weight approximately with respect to the total weight of the composition, without taking into account the propellant, and preferably between 6 and 15%.

20. Composition according to any one of the preceding claims, **characterized in that** the oxyalkylenated silicone or silicones are present in proportions of between 0.01 and 10% by weight approximately with respect to the total weight of the composition, without taking into account the propellant, and preferably between 0.05 and 5%.

21. Composition according to any one of the preceding claims, **characterized in that** the water-soluble solvent or solvents having a boiling point of less than 85°C are present in concentrations of between 30 and 60%, these concentrations being expressed by weight with respect to the total weight of the composition, without taking into account the propellant.

22. Composition according to any one of the preceding claims, **characterized in that** the propellant or propellants are present in concentrations of between 1 and 20% by weight with respect to the total weight of the composition in the pressurized state and preferably between 5 and 15% by weight.

23. Composition according to any one of the preceding claims, **characterized in that** it also contains surface-active agents, thickening agents, preservatives, sequestering agents, softeners, dyes, viscosity-modifying agents, foam-modifying agents, foam stabilizers, pearlescent agents, moisturizing agents, agents for combating dandruff, antiseborrhoeic agents, sunscreens, proteins, vitamins, plasticizers, hydroxy acids, electrolytes, fragrances and conditioning agents.

24. Cosmetic composition in the form of a foam, **characterized in that** it results from the expansion in the air of a composition as defined in any one of Claims 1 to 23.

25. Use of an oxyalkylenated silicone comprising at least one oxyalkylenated group of (-CₓH₂ₓO)ₐ type in which x varies from 2 to 6 and a is greater than or equal to 1 as foaming agent for compositions pressurized in an aerosol device which are capable of forming a foam at the outlet of the said device and which comprise at least one fixing polymer and at least 30% by weight of a solvent which is soluble in water to more than 5% and which has a boiling point of less than 85°C, this concentration being expressed with respect to the propellant-free composition.

26. Process for the cosmetic treatment of keratinous fibres, **characterized in that** a composition as defined in any one of Claims 1 to 24 is applied to keratinous fibres.
